# EUROPEAN PATENT SPECIFICATION

(11) **EP 3 891 121 B1**
(45) Date of publication and mention of the grant of the patent: **30.08.2023**
(21) Application number: 19892699.0
(22) Date of filing: 05.12.2019
(51) Int. Cl.: C07C 55/20, A23L 29/30, A23L 33/125, A61K 31/194, A61K 47/12

(54) **SEBACIC ACID COMPOSITIONS AND METHODS FOR TASTE MODULATION**
SEBACINSÄUREZUSAMMENSETZUNGEN UND VERFAHREN ZUR GESCHMACKSMODULATION
COMPOSITIONS D'ACIDE SÉBACIQUE ET PROCÉDÉS DE MODULATION DU GOÛT

(30) Priority: 06.12.2018 US 201862776150 P
(43) Date of publication of application: 13.10.2021
(73) Proprietor: International Flavors & Fragrances Inc., New York, NY 10019 (US)
(72) Inventor: WU, Hou, East Brunswick, NJ 08816 (US); HUBER, Michelle, Eve, Cream Ridge, NJ 08514 (US); JOHN, Thumpalasserill, V., Morganville, NJ 07751 (US); LI, Xiaodong, Middletown, NJ 07748 (US)
(74) Representative: Barker Brettell LLP
(86) International application number: PCT/US2019/064589
(87) International publication number: WO 2020/118003

(56) References cited:
- EP-A1- 0 259 996
- US-A- 4 906 455
- US-A1- 2016 235 102
- US-B2- 8 962 689
- ISIDOROV V.A. ET AL: "Gas chromatographic-mass spectrometric investigation of volatile and extractable compounds of crude royal jelly", JOURNAL OF CHROMATOGRAPHY B, vol. 885-886, 1 February 2012 (2012-02-01) , pages 109-116, XP055918596, NL ISSN: 1570-0232, DOI: 10.1016/j.jchromb.2011.12.025
- Brown William H.: "SOME CARBOXYLIC ACIDS PRESENT I N ROYAL JELLY'", , 1 January 1959 (1959-01-01), pages 2042-2046, XP055918591, Retrieved from the Internet: URL:https://cdnsciencepub.com/doi/pdf/10.1 139/v59-296 [retrieved on 2022-05-06]
- YAMAGA MASAYUKI ET AL: "Metabolism and pharmacokinetics of medium chain fatty acids after oral administration of royal jelly to healthy subjects", RSC ADVANCES, vol. 9, no. 27, 17 May 2019 (2019-05-17), pages 15392-15401, XP055918584, DOI: 10.1039/C9RA02991E Retrieved from the Internet: URL:https://pubs.rsc.org/en/content/articl epdf/2019/ra/c9ra02991e>
- MINGRONE et al.: "Use of dicarboxylic acids in type 2 diabetes", British Journal of Clinical Pharmacology, vol. 75, no. 3 13 January 2012 (2012-01-13), pages 671-676, XP055152877, Retrieved from the Internet: URL:https://bpspubs.ontinetibrary.witey.co m/doi/pdf/10.1111/j.1365-2125.2012.04177.x [retrieved on 2020-01-17]

## Description

### Introduction

This application claims benefit of priority to U.S. Provisional Patent Application Serial No. 62/776,150, filed December 6, 2018.

### Background

Sebacic acid is a white flake or powdered crystal slightly soluble in water. Sebacic acid and its derivatives such as azelaic acid have a variety of industrial uses as plasticizers, lubricants, hydraulic fluids, cosmetics, and candles. It is also used in the synthesis of polyamide and alkyd resins and as an intermediate for aromatics, antiseptics and painting materials. Isadorov et al. (2012, J. Chromatog., 885-886, p 109-116), Brown et al (Some Carboxylic acids present in royal jelly, 1959), US2016/235102A1, and US8,962,689B2 disclose compositions comprising a carbohydrate and sebacic acid.

### Summary of the Invention

In an aspect, the invention provides the use of sebacic acid in an amount effective to enhance the sweetness or mouthfeel of a carbohydrate-containing composition, wherein the effective amount is from 2 ppm to 1000 ppm. Also disclosed is a consumable composed of a carbohydrate sweetener or flavoring and sebacic acid, wherein the consumable may be a food product, pharmaceutical composition, a dietary supplement, a nutraceutical, a dental hygienic composition, a tabletop sweetener, a beverage, or a cosmetic product. Also disclosed is a method for enhancing the sweetness or mouthfeel of a consumable by adding sebacic acid to the consumable is also provided. The compositions and method may further include the use of traumatic acid and/or an isomer thereof such as 3-dodecenedioic acid.

### Detailed Description of the Invention

It has now been found that sebacic acid exhibits a sweet enhancement effect as well as mouthfeel modifying functionalities. Accordingly, the present invention relates to the use sebacic acid as an additive to enhance the sweetness and mouthfeel of a consumable.

As is known in the art, sebacic acid or decanedioic acid (CAS No. 111-20-6) is a linear, saturated dicarboxylic acid. Sebacic acid of use in this invention may be obtained from a natural source or prepared by synthetic methods. Natural sources of sebacic acid include royal jelly (Moutsatsou, et al. (2010) PLoS ONE 5: e15594), *Boehmeria nivea, Caesalpinia pulcherrima* and *Agave americana* (Matic (1956) Biochem. J. 63(1) :168-176). Sebacic acid may be isolated from one or more of these natural sources by ether extraction and sequential alkalization, acidification and ether extraction (Brown & Freure (1959) Can. J. Chem. 37:2042-2046); or extraction with an aqueous solution at elevated temperatures (e.g., 50-100°C) followed by separation of the two immiscible phases and precipitation of the dicarboxylic acids by cooling to aqueous phase (US 2,798,093). Chromatographic fractionation typically includes column chromatography and may based on molecular sizing, charge, solubility and/or polarity. Depending on the type of chromatographic method, column chromatography can be carried out with matrix materials composed of, for example, dextran, agarose, polyacrylamide or silica and can include solvents such as dimethyl sulfoxide, pyridine, water, dimethylformamide, methanol, saline, ethylene dichloride, chloroform, propanol, ethanol, isobutanol, formamide, methylene dichloride, butanol, acetonitrile, isopropanol, tetrahydrofuran, dioxane, chloroform/dichloromethane, etc. Typically, the product of the chromatographic step is collected in multiple fractions, which may then be tested for the presence of the desired compound using any suitable analytical technique (e.g., thin layer chromatography, mass spectrometry). Fractions enriched in the desired compound may then be selected for further purification.

Alternatively, sebacic acid may be synthesized. For example, sebacic acid can be synthesized by castor oil electrooxidation (US 4,237,317), which produces high purity sebacic acid from readily available adipic acid. The process includes the steps of partially esterifying adipic acid to form monomethyl adipate, subjecting the potassium salt of monomethyl adipate in a mixture of methanol and water to electrolysis to give dimethyl sebacate, and hydrolyzing the dimethyl sebacate to sebacic acid. In yet other embodiments, sebacic acid is obtained from a commercial source such as Cayman Chemical (Ann Arbor, MI) or Sigma-Aldrich (St. Louis, MO). In particular embodiments, the sebacic acid is purified to at least 95%, 96%, 97%, 98%, 99% or 100% homogeneity.

While salts of sebacic acid may be used in this invention, preferably the sebacic acid is not derivatized or conjugated to another molecule, i.e., the sebacic acid is unconjugated sebacic acid.

As described herein, sebacic acid enhances the taste and flavor of consumables. The taste and flavor profile of a consumable including sebacic acid may be enhanced or more intense (e.g., by 5%, 10%, 20%, 30% or more intense) than a comparative taste and flavor profile of a comparative consumable which does not include sebacic acid as an additive. Further, the mouthfeel of a consumable including sebacic acid may be improved in relation to the mouthfeel of a comparative consumable that does not include sebacic acid.

In particular embodiments, sebacic acid enhances the sweetness and mouthfeel of a consumable containing a carbohydrate sweetener or flavoring. As used herein, the term "sweetness" or "sweetness intensity" refers to the relative strength of sweet sensation as observed or experienced by an individual, *e*.*g*., a human, or a degree or amount of sweetness detected by a taster, for example on the scale from 0 (none) to 8 (very strong) used in sensory evaluations according to the procedure described in American Society for Testing Materials, Special Technical Publication-434: "Manual on Sensory Testing Methods," ASTM International, West Conshohocken, Pa. (1996). The mouthfeel of a substance relates to the physical sensations in the mouth produced by a particular food. By way of illustration, a "sugary mouthfeel" is the physical sensation observed or experienced by an individual, *e*.*g*., a human, upon consumption of a sugar. In accordance with the present invention, sebacic acid enhances at least sweet/sugary mouthfeel and optionally fatty mouthfeel.

As used herein, a consumable includes all food products, pharmaceutical compositions, dietary supplements, nutraceuticals, dental hygienic compositions, tabletop sweeteners, beverages, or cosmetic products. The consumable may include one or more carbohydrate sweeteners or flavorings. The carbohydrate sweetener or flavoring can be present in the consumable inherently (e.g., in food products containing fruits) or the carbohydrate sweetener or flavoring is added into the consumable (i.e., an exogenous sweetener or flavoring). Suitable carbohydrate sweeteners of the present disclosure include, but are not limited to, sucrose, fructose, glucose, high fructose corn syrup (containing fructose and glucose), xylose, arabinose, rhamnose, and sugar alcohols, such as erythritol, xylitol, mannitol, sorbitol, or inositol. The carbohydrate sweetener may be sucrose, fructose, glucose, high fructose corn syrup, xylose, arabinose or rhamnose, preferably sucrose, fructose, or glucose. The carbohydrate sweetener may be sucrose. The carbohydrate sweetener may be glucose. The carbohydrate sweetener may be fructose. The carbohydrate sweetener may be a sugar alcohol, *e*.*g*., erythritol, xylitol, mannitol, sorbitol, or inositol.

Flavorings of use in this disclosure include, but are not limited to, Natural Sweet Flavor #2 (WO 2012/129451), stevioside, rebaudioside A, rebaudioside B, rebaudioside C, rebaudioside D, rebaudioside E, rebaudioside F, dulcoside A, dulcoside B, stevia, alpha-glucosyl stevia, fructosyl stevia, galactosyl stevia, beta-glucosyl stevia, siamenoside, mogroside IV, mogroside V, Luo Han Guo sweetener, monatin and its salts, glycyrrhizic acid and its salts (*e*.*g*., as found in MAGNASWEET), curculin, thaumatin, monellin, mabinlin, brazzein, hernandulcin, phyllodulcin, glycyphyllin, phloridzin, trilobtain, baiyunoside, osladin, polypodoside A, pterocaryoside A, pterocaryoside B, mukurozioside, phlomisoside I, periandrin I, abrusoside A, cyclocarioside I, or a combination thereof. The flavoring may be Natural Sweet Flavor #2 (also known as NSF-02), which contains glucosylated steviol glycosides and dextrin.

When added to a consumable as an additive, sebacic acid is used in an amount effective to enhance the sweetness or mouthfeel of a carbohydrate sweetener or flavoring without exhibiting any off-taste. Any amount of sebacic acid that provides the desired degree of sweetness or mouthfeel enhancement can be used. Preferably, the amount of sebacic acid present in the consumable is an amount as low as 0.05 ppm, in an amount as low as 0.2 ppm, in an amount as low as 1 ppm, or in an amount as low as 10 ppm. The sebacic acid can be included in the consumable in an amount that is as high as 1000 ppm, in an amount as high as 500 ppm, or in an amount as high as 100 ppm. The sebacic acid may further be present within any range delimited by any pair of the foregoing values, such as between 0.2 ppm and 1000 ppm, or between 1 ppm and 100 ppm, for example. The term "ppm" as used herein means part per million by weight or volume, for example, the weight of the component (in milligrams) per liter of solution, *i*.*e*., µg/ml.

In some embodiments, the invention embraces a combination of sebacic acid and traumatic acid (CAS No. 6402-36-4) and/or its isomer, 3-dodecenedioic acid (CAS No. 189034-80-8. In particular embodiments, the weight ratio of traumatic acid (or its isomer) to sebacic acid is at least 1:1, 2:1, 3:1, 4:1, 5:1, 6:1, 7:1, 8:1, or 9:1.

The phrase "food product" as used herein includes, but is not limited to, fruits, vegetables, juices, meat products (*e*.*g*., ham, bacon and sausage), egg products, fruit concentrates, gelatins and gelatin-like products (*e*.*g*., jams, jellies, preserves, and the like) milk products (*e*.*g*., ice cream, sour cream and sherbet), icings, syrups including molasses, corn products, wheat products, rye products, soybean products, oat products, rice products and barley products, nut meats and nut products, cakes, cookies, confectionaries (*e*.*g*., candies, gums, fruit flavored drops, and chocolates), chewing gum, mints, creams, ice cream, pies and breads, and beverages such as coffee, tea, carbonated soft drinks (e.g., soft drinks sold under the tradenames COKE^{®} and PEPSI^{®}), non-carbonated soft drinks, juices and other fruit drinks, sports drinks such as those sold under the tradename GATORADE^{®}, alcoholic beverages, such as beers, wines and liquors, and flavored drinks sold under the tradename KOOL-AID^{®}. Food products also include condiments such as herbs, spices and seasonings, and flavor enhancers, such as monosodium glutamate. A food product also includes prepared packaged products, such as dietetic sweeteners, liquid sweeteners, granulated flavor mixes which upon reconstitution with water provide non-carbonated drinks, instant pudding mixes, instant coffee and tea, coffee whiteners, malted milk mixes, pet foods, livestock feed, tobacco, and materials for baking applications, such as powdered baking mixes for the preparation of breads, cookies, cakes, pancakes, donuts and the like. Food products also include diet or low-calorie food and beverages containing little or no sucrose. Especially preferred food products are carbonated beverages. Preferably, the consumable in which the sweetness or mouthfeel is enhanced contains a decreased level of the carbohydrate sweetener. For example, an improved carbonated soft drink can be produced with the same sweetness as the known carbonated soft drink but with a lower sugar content by adding sebacic acid.

The consumable can also be a pharmaceutical composition. Preferred compositions are pharmaceutical compositions containing sebacic acid and one or more pharmaceutically acceptable excipients. These pharmaceutical compositions can be used to formulate pharmaceutical drugs containing one or more active agents that exert a biological effect other than sweetness enhancement. The pharmaceutical composition preferably further includes one or more active agents that exert a biological effect. Such active agents include pharmaceutical and biological agents that have an activity other than taste enhancement. Such active agents are well known in the art. See, *e.g.,* The Physician's Desk Reference. Such compositions can be prepared according to procedures known in the art, for example, as described in Remington's Pharmaceutical Sciences, Mack Publishing Co., Easton, PA. Such an active agent may include bronchodilators, anorexiants, antihistamines, nutritional supplements, laxatives, analgesics, anesthetics, antacids, H₂-receptor antagonists, anticholinergics, antidiarrheals, demulcents, antitussives, antinauseants, antimicrobials, antibacterials, antifungals, antivirals, expectorants, anti-inflammatory agents, antipyretics, and mixtures thereof. The active agent may be a antipyretic or analgesic, *e*.*g*., ibuprofen, acetaminophen, or aspirin; laxative, *e.g.,* phenolphthalein dioctyl sodium sulfosuccinate; appetite depressant, *e.g.,* amphetamine, phenylpropanolamine, phenylpropanolamine hydrochloride, or caffeine; antacidic, *e.g.,* calcium carbonate; antiasthmatic, *e.g.,* theophylline; antidiuretic, *e.g.,* diphenoxylate hydrochloride; agent active against flatulence, *e.g.,* simethecon; migraine agent, *e.g.,* ergotaminetartrate; psychopharmacological agent, *e.g.,* haloperidol; spasmolytic or sedative, *e*.*g*., phenobarbitol; antihyperkinetic, *e.g.,* methyldopa or methylphenidate; tranquilizer, *e*.*g*., a benzodiazepine, hydroxinmeprobramate or phenothiazine; antihistaminic, *e.g.,* astemizol, chloropheniramine maleate, pyridamine maleate, doxlamine succinate, bromopheniramine maleate, phenyltoloxamine citrate, chlorocyclizine hydrochloride, pheniramine maleate, or phenindamine tartrate; decongestant, *e.g.,* phenylpropanolamine hydrochloride, phenylephrine hydrochloride, pseudoephedrine hydrochloride, pseudoephedrine sulfate, phenylpropanolamine bitartrate, or ephedrine; beta-receptor blocker, *e.g.,* propanolol; agent for alcohol withdrawal, *e*.*g*., disulfuram; antitussive, *e.g.,* benzocaine, dextromethorphan, dextromethorphan hydrobromide, noscapine, carbetapentane citrate, or chlophedianol hydrochloride; fluorine supplement, *e.g.,* sodium fluoride; local antibiotic, *e.g.,* tetracycline or cleocine; corticosteroid supplement, *e*.*g*., prednisone or prednisolone; agent against goiter formation, *e.g.,* colchicine or allopurinol; antiepileptic, *e*.*g*., phenyloine sodium; agent against dehydration, *e.g.,* electrolyte supplement; antiseptic, *e.g.,* cetylpyridinium chloride; NSAID, *e*.*g*., acetaminophen, ibuprofen, naproxen, or salt thereof; gastrointestinal active agent, *e.g.,* loperamide and famotidine; an alkaloid, *e*.*g*., codeine phosphate, codeine sulfate, or morphine; supplement for a trace element, *e*.*g*., sodium chloride, zinc chloride, calcium carbonate, magnesium oxide, or other alkali metal salt or alkali earth metal salt; vitamin; ion-exchange resin, *e*.*g*., cholestyramine; cholesterol-depressant or lipid-lowering substance; antiarrhythmic, *e*.*g*., N-acetylprocainamide; or expectorant, *e*.*g*., guaifenesin.

The consumable may be a dietary supplement or nutraceutical. Examples of such compositions having an undesirable taste include, but are not limited to, enteral nutrition products for treatment of nutritional deficit, trauma, surgery, Crohn's disease, renal disease, hypertension, obesity and the like, to promote athletic performance, muscle enhancement or general well-being or inborn errors of metabolism such as phenylketonuria. In particular, such compositions can contain one or more amino acids which have a bitter or metallic taste or aftertaste. Such amino acids include, but are not limited to, essential amino acids such as L isomers of leucine, isoleucine, histidine, lysine, methionine, phenylalanine, threonine, tryptophan, tyrosine, and valine.

The consumable of the present disclosure may be a dental hygienic composition, containing a carbohydrate sweetener or flavoring and sebacic acid. Dental hygienic compositions are known in the art and include, but are not necessarily limited to, toothpaste, mouthwash, plaque rinse, dental floss, dental pain relievers (such as ANBESOL^{™}), and the like. The dental hygienic composition may include one carbohydrate sweetener. The dental hygienic composition may include more than one carbohydrate sweetener. The dental hygienic composition may include sucrose and corn syrup, or sucrose and aspartame.

The consumable of the present disclosure may be a cosmetic product containing a carbohydrate sweetener or flavoring and sebacic acid. For example, but not by way of limitation, the cosmetic product can be a face cream, lipstick, lip gloss, and the like. Other suitable compositions of the disclosure include lip balm, such as lip balm sold under the tradename CHAPSTICK^{®} or BURT'S BEESWAX^{®}.

When used in the methods and compositions of this disclosure, sebacic acid is added to a consumable (i.e., the sebacic acid is not an endogenous component of the consumable) in an amount effective to enhance the sweetness or mouthfeel of the consumable including a carbohydrate sweetener or flavoring. In this respect, the amount of carbohydrate sweetener or flavoring added to the consumable may be reduced while retaining the desired sweetness level. Thus, the present dislcosure also provides methods and compositions for enhancing the sweetness and mouthfeel of a carbohydrate sweetener and/or flavoring and decreasing the amount of a carbohydrate sweetener and/or flavoring in a consumable by combining the carbohydrate sweetener and/or flavoring with sebacic acid. The carbohydrate sweetener or flavoring and sebacic acid may be used at a 10000:1, 1000:1, or 100:1 ratio. The disclosure provides a consumable containing an effective amount of a combination of sebacic acid and a carbohydrate sweetener or flavoring in a reduced amount in order to achieve the same level of sweetness when the carbohydrate sweetener or flavoring is used alone in the traditional amount. In this respect, the amount of carbohydrate sweetener or flavoring used in a consumable can be reduced by at least about 5%, 10%, 20%, or 30%. Unless otherwise specified, percentages (%) are by weight.

The invention is described in greater detail by the following non-limiting examples.

### Example 1: Sensory Evaluation of Sebacic Acid at Different Amounts in Sugar Water

Sugar water (4% sucrose in drinking water) was prepared and used as a base solution. Different amounts of sebacic acid were added to the base solution and descriptive sensory evaluations were performed (Table 1).

**TABLE 1**

| Sebacic acid (ppm) | Taste Evaluation |
|---|---|
| 0.02 | Slight sweet and mouthfeel enhancement, barely detectable. |
| 1000 | Strong sweet and mouthfeel enhancement with strong chemical off-taste |

### Example 2: Taste Modulation Using Sebacic Acid

Descriptive sensory evaluations were carried out using different amounts of sebacic acid in different applications. The results of this analysis, as compared to the same applications in the absence of sebacic acid, are presented in Table 2.

**TABLE 2**

| Application | Sebacic Acid | Taste Evaluation |
|---|---|---|
| Water | 40 ppm | Fatty and oily |
| 3% Sugar + 0.05% Citric Acid Water Solution | 40 ppm | Fatty and oily |
| 3% Sugar-Sweetened Full Fat Milk | 7.5 ppm | Increases fatty perception and enhances mouthfeel |
| Strawberry Flavored Yogurt (5% Sugar, 0.4% Fat, 8.7 Grams Protein) | 15 ppm | Sweet up front, more strawberry, cuts acid |
| Mixed Berry Yogurt Smoothie (1% Fat, 9% Sugar, 3% Protein) | 7.5 ppm | Cuts sour end, increased sweet milk notes |

### Example 3: Sensory Evaluation of Sebacic Acid and Traumatic Acid in Sweetened Milk

Sweetened milk (2% fat milk with 2% sucrose) was prepared and used as a base solution ("base"). Sebacic acid and traumatic acid were added to the base solution and descriptive sensory evaluations were performed (Table 3)

**TABLE 3**

| Sample (ppm) | Taste Evaluation | Strength |
|---|---|---|
| Base (N/A) | Sweetened milk taste | + |
| Sebacic Acid (2 ppm) | Sweeter and more mouthfeel than the base. More overall dairy impression and more dairy fat aroma | ++ |
| Traumatic Acid (2 ppm) | Sweeter and more mouthfeel than the base, slightly coconutty, and waxy. More overall dairy impression and more dairy fat aroma | +++ |
| Traumatic Acid (2 ppm) and Sebacic Acid (2 ppm) | Sweeter and more mouthfeel than the base. More overall dairy impression and more dairy fat aroma | ++++ |

The results of this analysis indicated that sebacic acid (2 ppm) and traumatic acid (2 ppm) each increased sweetness and mouthfeel of sweetened milk. When used together, sebacic acid (2 ppm) and traumatic acid (2 ppm) provided the most increase in sweetness and mouthfeel.

In addition, the sweetness and mouthfeel enhancement as well as off-flavor masking of sebacic acid was compared with a series of analogs including adipic acid, pimelic acid, suberic acid, undecanedioic acid, dodecanedioic acid, 1,11-undecanedicarboxylic acid, tetradecanedioic acid and hexadecanedioic acid at 2 ppm. Among all the compounds tested, sebacic acid exhibited the strongest and the longest-lasting effect, which was significantly superior to all other compounds.

## Claims

1. Use of sebacic acid in an amount effective to enhance the sweetness or mouthfeel of a carbohydrate-containing composition , wherein the effective amount is from 2 ppm to 1000 ppm .

2. The use of claim 1, further comprising adding traumatic acid, 3-dodecenedioic acid, or a combination thereof to the composition.

3. The use of claim 1 or claim 2, wherein the carbohydrate-containing composition is a food product, pharmaceutical composition, a dietary supplement, a nutraceutical, a dental hygienic composition, a tabletop sweetener, a beverage, or a cosmetic product.

## Patentansprüche

1. Verwendung von Sebacinsäure in einer Menge, die wirksam ist, die Süße oder das Mundgefühl einer kohlenhydrathaltigen Zusammensetzung zu verstärken, wobei die wirksame Menge von 2 ppm bis 1000 ppm beträgt.

2. Verwendung gemäß Anspruch 1, ferner umfassend Zugeben von Traumatinsäure, 3-Dodecendisäure oder einer Kombination davon zu der Zusammensetzung.

3. Verwendung gemäß Anspruch 1 oder Anspruch 2, wobei die kohlenhydrathaltige Zusammensetzung ein Lebensmittelprodukt, eine pharmazeutische Zusammensetzung, eine Nahrungsergänzung, ein Nutrazeutikum, eine Zahnhygienezusammensetzung, ein Tafelsüßungsmittel, ein Getränk oder ein kosmetisches Produkt ist.

## Revendications

1. Utilisation d'acide sébacique selon une quantité efficace pour améliorer la sucrosité ou la sensation en bouche d'une composition contenant des glucides, la quantité efficace allant de 2 ppm à 1000 ppm.

2. Utilisation selon la revendication 1, comprenant en outre l'addition d'acide traumatique, d'acide 3-dodécènedioïque, ou d'une combinaison de ceux-ci, à la composition.

3. Utilisation selon la revendication 1 ou la revendication 2, la composition contenant des glucides étant un produit alimentaire, une composition pharmaceutique, un complément alimentaire, un nutraceutique, une composition d'hygiène dentaire, un édulcorant de table, une boisson, ou un produit cosmétique.
